# EUROPÄISCHE PATENTSCHRIFT

(11) **EP 3 923 838 B1**
(45) Veröffentlichungstag und Bekanntmachung des Hinweises auf die Patenterteilung: **05.04.2023**
(21) Anmeldenummer: 20707357.8
(22) Anmeldetag: 11.02.2020
(51) Int. Cl.: A61B 17/66

(54) **SYSTEM ZUR MODIFIKATION EINES MENSCHLICHEN ODER TIERISCHEN KNOCHENS**
SYSTEM FOR MODIFYING A HUMAN OR ANIMAL BONE
SYSTÈME DE MODIFICATION D'UN OS HUMAIN OU ANIMAL

(30) Priorität: 12.02.2019 DE 102019201825
(43) Veröffentlichungstag der Anmeldung: 22.12.2021
(73) Patentinhaber: Zyfoma GmbH, 64331 Weiterstadt (DE)
(72) Erfinder: SCHLEE, Markus, 91301 Forchheim (DE)
(74) Vertreter: Tergau & Walkenhorst Patentanwälte PartGmbB
(86) Internationale Anmeldenummer: PCT/EP2020/053439
(87) Internationale Veröffentlichungsnummer: WO 2020/165151

(56) Entgegenhaltungen:
- US-A1- 2003 104 339
- US-A1- 2005 159 754

## Beschreibung

Die Erfindung betrifft ein System zur Modifikation eines menschlichen oder tierischen Knochens.

Insbesondere im Bereich der plastischen oder dentalen Chirurgie kann das Bedürfnis einer gezielten Modifikation des vorhandenen Knochens bestehen. Dabei können sowohl additive Modifikationen, bei denen der vorhandenen Knochenmasse weitere Knochenmasse hinzugefügt wird, als auch subtraktive Modifikationen, bei denen die vorhandene Knochenmasse reduziert wird, wünschenswert sein.

Additive Modifikationen im Sinne eines Knochenaufbaus können insbesondere im Bereich der Traumatologie, plastischen Chirurgie oder Implantologie, beispielsweise der dentalen Implantologie, notwendig oder wünschenswert sein, um eine für eine sichere Verankerung eines Implantats in ausreichendem Knochenvolumen mit ausreichend hoher Primärstabilität ausreichende Knochensubstanz bereitstellen zu können. Ein Knochenaufbau wird nämlich in der Regel immer dann notwendig, wenn die Menge an vorhandenem Knochen für die Implantation nicht ausreicht oder die Kieferknochenstruktur nicht stimmt, um das Zahnimplantat fest im Kiefer zu verankern. Grund hierfür kann beispielsweise Knochenschwund oder Knochenabbau sein, der in Folge von Infektionen, Zahnlosigkeit oder Zahnerkrankungen entstanden ist und ohne eine adäquate Behandlung weiter fortschreitet. Dann ist auch das übrige Gebiss von schweren Folgen für die Zahngesundheit und weiterem Zahnverlust bedroht. Im Allgemeinen wird in der Chirurgie fehlender Knochen aufgebaut oder überschüssiger Knochen entfernt; falls dies erforderlich ist oder gewünscht wird. Knochenaufbau kann beispielsweise nach Unfall, Tumorentfernung, Zahnverlust oder in der kosmetischen, bzw. plastischen Chirurgie erforderlich sein. Die Reduktion von Knochen kann beispielsweise in der plastischen Chirurgie (Nasen-, Wangenknochenkorrektur, etc.) oder in der orthognathen Chirurgie sinnvoll sein.

Falls ein Knochenaufbau erforderlich werden sollte, kann das Knochenvolumen in der Art einer Transplantation autogenen Knochens vermehrt werden, indem andernorts Knochen entnommen und an der Empfängerstelle wieder eingesetzt wird. Nachteilig ist bei einer solchen Methode, dass zwei Operationen am Patienten durchgeführt werden müssen und sich dadurch das Komplikationsrisiko erhöht. Alternativ kann das Knochenvolumen aber auch durch Zuführung von so genanntem Knochenersatzmaterial vermehrt werden. Dabei werden üblicherweise Implantate aus Knochenersatzmaterialien (menschlichem oder tierischem Spenderknochen oder synthetischen Materialien, insbesondere alloplastischem Material) hergestellt und in den aufzubauenden Bereich eingebracht. Allogener Spenderknochen birgt jedoch ein immunologisches und/oder ein Infektionsrisiko in sich. Die verfügbare Menge an Spendermaterial ist zudem aus ethischen und juristischen Gründen limitiert. Beim alternativ möglichen Interspeziestransfer von Biomaterialien (Materialien tierischen Ursprunges, xenogenes Knochenersatzmaterial) kann es immunologische Probleme und Limitationen geben, die aus strukturellen oder anatomischen Unterschieden entstehen. In der Regel wird implantiertes xenogenes Knochenersatzmaterial zumindest für lange Zeit im Knochenregenerat oder sogar nur bindegewebig eingeschieden Die biologische Potenz zur Knochnregeneration solcher Materialien ist limitiert.

Im Vergleich zum autogenen Knochentransplantat entstehen somit im Ergebnis einer Implantation synthetischer Knochenersatzmaterialien Substitute, die ausnahmslos und vor allem bei den keramischen Implantaten als unvollständige Restitution zu werten sind. Ein ideales Knochensubstitut gibt es derzeit nicht.

Dokument US 2005/159754 A1 offenbart ein System zur Modifikation eines menschlichen Knochens mit einem Hub-/Druckelement.

Der Erfindung liegt daher die Aufgabe zugrunde, ein System zur Modifikation eines menschlichen oder tierischen Knochens anzugeben, mit dem auf besonders einfache und insbesondere auch besonders gut verträgliche Weise zuverlässig ein Knochenaufbau oder eine Knochenreduktion erreicht werden kann.

Diese Aufgabe wird erfindungsgemäß gelöst mit einem zur Einbringung zwischen die Knochenhaut des zu behandelnden Knochens und den darunter liegenden Knochen oder das darüberliegende Bindegewebe vorgesehenen flächig ausgedehnten Hub-/Druckelement, und mit einem diesem zugeordneten Steuerelement, wobei das Steuerelement zur Ausübung einer magnetischen Druck- oder Zugkraft auf das Hub-/Druckelement ausgelegt ist.

Die Erfindung geht dabei von der Überlegung aus, dass für eine besonders gut verträgliche und auch zuverlässige Modifikation von Knochenvolumen auf die natürlichen Mechanismen der Knochenbildung und des Knochenwachstums zurückgegriffen werden sollte. Die natürliche Bildung von Knochen, oder die Remodellation oder die Heilung einer Knochenverletzung, erfolgt durch Osteoblasten. Diese knochenbildenden Zellen und deren Vorläufer finden sich in der Knochenhaut (Periost), um die Blutgefäße (Perizyten), in der inneren Knochenhaut (Endost) und im Knochen selbst. Bei expandiertem Knochengewebe, beispielsweise bei Spaltbildung infolge von Knochenbrüchen, wandern aus den Gefäßwänden des expandierten Gewebes Perizyten aus, die sich zu Osteoprogenitorzellen und später zu Osteoblasten entwickeln. Gefäßanastomosen aus dem Knochen (Volkmannsche Gefäße) sind mit Gefäßen im Stratum fibrosum des Periosts verbunden. Die Osteoprogenitorzellen oder Osteoblasten im Kambium des Periosts sind ebenso an der Synthese neuen Knochens beteiligt. Daher führen Deformationen des Periosts, speziell die graduelle Distraktion von der Knochenoberfläche, zur Knochenneubildung (Knochenauflagerung) an einer bestehenden Knochenoberfläche.

Unter gezielter Nutzung dieser Erkenntnisse ist nunmehr vorgesehen, zum Zwecke des Knochenaufbaus gezielt und einstellbar einen Freiraum oberhalb des Knochens und unterhalb des Periosts oder der Knochenhaut zu schaffen, in den die Perzyten einwandern und nachfolgend eine Knochenneubildung bewirken können. Um den Freiraum zu schaffen, wurden in der Vergangenheit Metallplatten oder Gitter eingebracht. Das Weichgewebe wurde durch das Weichgewebe perforierende Metallteile mechanisch vom Knochen abgehoben. Dieses Verfahren birgt durch die Perforation ein Infektionsrisiko, und die gewünschte Form des Knochens lässt sich nur ungenügend regenerieren. Demgegenüber ist nunmehr zur Schaffung des genannten Freiraums erfindungsgemäß ein flächig ausgeführtes Hub- oder Druckelement vorgesehen, das für die Hubbewegung zwischen Knochen und Periost eingeschoben wird und dann durch eine Krafteinwirkung von außen das Periost relativ zum Knochen anhebt. Dadurch wird der besagte Freiraum gezielt gebildet, und der Knochenaufbau kann stattfinden.

Im umgekehrten Fall, also bei angestrebter subtraktiver Modifikation mit gezieltem Knochenabbau, kann eine Krafteinwirkung auf das Druckelement in entgegengesetzter Richtung, also zum Knochen hin, vorgegeben werden, durch die Druck auf das Periost ausgeübt wird. In diesem Fall wird das Druckelement zwischen Periost und umgebendes oder darüberliegendes Bindegewebe eingeschoben. In Reaktion auf diesen Druck weicht die Knochensubtsanz zurück, so dass die in diesem Falle gewünschte lokale Reduktion des Knochenvolumens erreicht wird.

Um diese flächige Krafteinwirkung über das Hub-/Druckelement besonders schnonend und für den Patienten verträglich zu bewirken, ist die Kraftbeaufschlagung des Hub-/Druckelements durch ein zugeordnetes externes Steuergerät auf kontaktlose Weise, besonders bevorzugt magnetisch, ausgestaltet. Dabei kann das Steuergerät einen geeignet gewählten Magneten umfassen, und/oder das Hub-/ Druckelement geeignet magnetisch ausgestaltet sein, so dass durch die magnetische Wechselwirkung die gewünschte kraftseitige Kopplung erreicht wird. Die kraftseitige Kopplung wird dabei insbesondere dadurch erreicht, dass das vom Steuergerät erzeugte Magnetfeld auf die Magnetisierung des Hub-/Druckelements und/oder dessen magnetisches Moment einwirkt und damit die erwünschte Magnetkraft erzeugt wird.

Vorteilhafte Ausgestaltungen der Erfindung sind Gegenstand der Unteransprüche.

Zum Zwecke des Knochenaufbaues wird das Hub-/Druckelement vorteilhafterweise temporär zwischen Knochen und Periost bzw. zwischen Periost und Bindegewebe implantiert. Hinsichtlich seiner Eigenschaften wie bespielsweise Flexibilität, Dicke, Durchlässigkeit oder dergleichen ist das Hub-/Druckelement vorteilhafterweise derart ausgestaltet, dass durch eine adäquate Permeabilität die Reanastomosierung des Periosts mit Gefäßen im Knochen ermöglicht ist. Andererseits sollte das Hub-/Druckelement mechanisch stabil genug ausgeführt sein, um Kräfte auf das Periost übertragen zu können. Es sollte zudem möglichst dünn sein, um die Deckung mir dem Hautlappen zu erleichtern und die Chirurgie beim Einbringen einfacher zu machen. Nach adäquater Einheilzeit wird das Hub-/Druckelement durch konstant oder periodisch wirkende Kräfte, beispielsweise durch magnetische Kräfte, zunehmend vom Knochen separiert. Dadurch kommt es zur Auflagerung und Neubildung von Knochen. Durch die Steuerung der Zugrichtung der Kräfte, deren Intensität und Dauer kann die Form des Knochens vorherbestimmt oder beeinflusst werden.

Zum Zwecke des Knochenabbaues wird das Druckelement temporär zwischen Periost und darüberliegendem Bindegewebe implantiert. Nach adäquater Einheilzeit übt das Druckelement durch gesteuerte Kräfte Druck auf Periost und Knochen aus. Es kommt dadurch zu einem Knochenabbau. Durch die Steuerung der Druckrichtung der Kräfte, deren Intensität und Dauer kann die Form des Knochens vorherbestimmt werden.

Besonders bevorzugt ist das Hub-/Druckelement unter Berücksichtigung folgender Kriterien und Auslegungsziele ausgestaltet:
1. Es sollte so dünn wie möglich ausgeführt sein. Damit ist sichergestellt, dass nach Freilegung des Knochens und Applikation des Hub-/Druckelements das Periost und das darüberliegende Weichgewebe spannungsfrei über dem Hub-/Druckelement geschlossen werden kann. Dies ist für die Wundheilung wichtig. Andererseits ist hier eine Grenze aber auch eine ausreichende mechanische Stabilität, um Kräfte übertragen zu können.
2. Es sollte flexibel, d. h. in der Form veränderbar, sein, um sich bei Knochenaufbau von "klein nach groß" verändern zu können und analog bei Knochenabbau umgekehrt.
3. Es sollte bevorzugt perforiert oder auf andere Weise in der Fläche durchlässig ausgeführt sein, um einen Gefäßneuanschluß zwischen Gefäßen im Knochen und Periost zu ermöglichen. Vorteilhafterweise ist ein Verhältnis Perforation zu Fläche von mindestens 10 %, besonders bevorzugt mindestens 30 %, vorgesehen. Die Porengröße der die Perforation bildenden Poren ist dabei bevorzugt klein genug gewählt, um ein "Durchrutschen" des Periosts zu vermeiden und eine gleichmäßige Elevation des gesamten Weichgewebes zu erreichen.

Das zugeordnete Steuerelement hingegen ist vorzugsweise im Hinblick auf die vorgesehene magnetische Kraftübertragung geeignet zur Erzeugung eines Magnetfelds im Bereich der vorgesehenen Knochenmodifikation ausgelegt. Dazu kann das Steuerelement eine Anzahl von geeignet gewählten und positionierten Permanentmagneten und/oder einen geeignet ansteuerbaren Elektromagneten umfassen. Das Steuerelement ist bevorzugt für eine Positionierung außerhalb des
Patientenkörpers vorgesehen und ausgelegt; beispielsweise könnte es im Falle einer beabsichtigten Modifikation eines Kieferknochens durch eine Gesichtsmaske, einen Gurt, eine Schiene oder dergleichen außen am Gesicht des Patienten im Kieferbereich befestigbar sein.

Das System, umfassend das Hub-/Druckelement und das zugeordnete Steuerelement, ist vorzugsweise hinsichtlich der magnetischen Kopplung und Kraftübertragung geeignet für die angestrebte Schaffung des erwünschten Freiraums zwischen Periost und Knochen bzw. zwischen Periost und Bindegewebe ausgelegt. Dazu sollte die vom Steuerelement magnetisch auf das Hub-/Druckelement übertragene Kraft in etwa derart gewählt sein, dass pro Tag die sich ergebende Knochendistraktion etwa 0,5 mm beträgt. Um dies zu ermöglichen, weist das Hub-/ Druckelement vorteilhafterweise eine hierfür geeignet gewählte Magnetisierung oder Magnetisierbarkeit auf.

Das Hub- oder Druckelement kann expandierbar hergestellt werden, beispielsweise als Gitter, Strick-, Web- oder Häkelstoff. Das zugeordnete zweite magnetische Teil oder Steuerelement ist vorzugsweise beispielsweise in einer Schiene angebracht, die die gewünschte Form des Knochens abbildet. Dies ermöglicht eine gezielte, geführte Regeneration natürlichen Knochens bis zum gewünschten Volumen.

Besonders bevorzugt ist das Hub-/Druckelement permanent- oder ferromagnetisch.

Das Hub-/Druckelement kann eine über seine Fläche gesehen im Wesentlichen konstante Magnetisierung bzw. Magnetisierbarkeit aufweisen. Um allerdings einen Gefäßneuanschluß zwischen den Gefäßen im Knochen und dem Periost zu ermöglichen und damit die Knochenneubildung besonders zu begünstigen, ist das Hub-/Druckelement besonders bevorzugt perforiert oder auf andere Weise in der Fläche durchlässig ausgeführt. Damit einhergehend weist das Hub-/Druckelement vorteilhafterweise auch eine über seine Fläche gesehen in der Art einer Gitterstruktur verteilte Magnetisierung bzw. Magnetisierbarkeit auf.

Zur Bereitstellung der vorgesehenen magnetischen oder magnetisierbaren Eigenschaften weist das Hub-/Druckelement vorzugsweise Bestandteile aus einem geeignet gewählten Material, vorzugsweise einem magnetischen oder magnetisierbaren Metall, besonders bevorzugt Eisen, in einer geeigneten Form, beispielsweise als Draht, Flocken, Plättchen oder Partikel, auf. Im Sinne einer besonders guten Biokompatibiität oder Verträglichkeit sind diese Bestandteile vorzugsweise in der Art einer gekapselten Ausführung geeignet von einem biokompatiblen Material, insbesondere einem Kunststoff, umgeben und vollständig in diesen eingebettet. Auf besonders einfache Weise und zudem noch besonders nutzungsfreundlich und flexibel ist dies erreichbar, indem das Hub-/Druckelement in ganz besonders bevorzugter Ausgestaltung von einer Kunststoffmatrix gebildet ist, in die magnetische oder magnetisierbare Partikel eingebettet sind. Besonders bevorzugt sind als Materialien hierfür Teflon, Polypropylen oder andere Materialien, aus denen Fäden für chirurgische Anwendungen hergestellt werden, vorgesehen.

Die mit der Erfindung erzielten Vorteile bestehen insbesondere darin, dass durch das von außen mit einer geeigneten Kraft beaufschlagbare, zur Einbringung zwischen Periost und Knochen bzw. zwischen Periost und Bindegewebe vorgesehene Hub-/Druckelement eine Modifikation des zu behandelnden Knochens auf besonders schonende und den natürlichen Mechanismen nahe kommende Weise erreicht werden kann. Damit ist die angestrebte Knochenmodifikation auf besonders schonende und für den Patienten besonders gut verträgliche Weise erreichbar.

Ein Ausführungsbeispiel der Erfindung wird anhand einer Zeichnung näher erläutert. Darin zeigen:
- FIG. 1: ein System zur Modifikation eines menschlichen oder tierischen Knochens,
- FIG. 2: ein Hub-/Druckelement des Systems nach FIG. 1 ausschnittsweise im Querschnitt, und
- FIG. 3: das Hub-/Druckelement nach FIG. 2 in Draufsicht.

Gleiche Teile sind in allen Figuren mit denselben Bezugszeichen versehen.

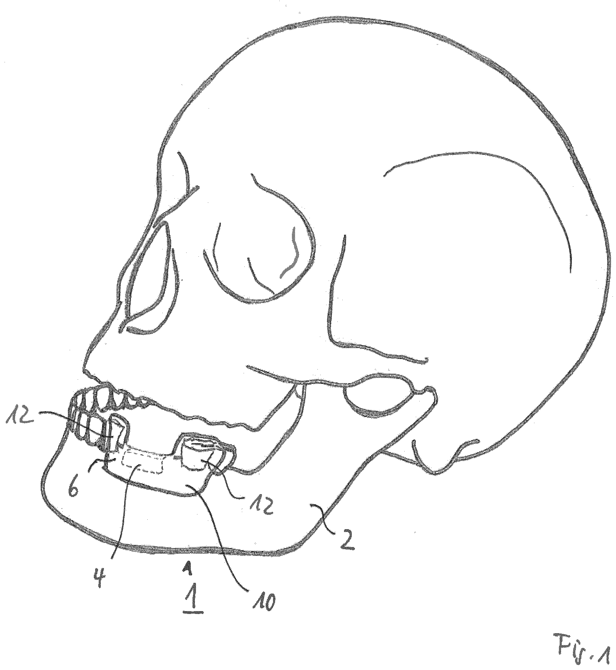

Das System 1 gemäß FIG. 1 ist zur gezielten Modifikation eines menschlichen oder tierischen Knochens auf besonders schonende und für den Patienten verträgliche Weise ausgeführt. Im Ausführungsbeispiel gem. FIG. 1 ist dies für die Behandlung des Kieferknochens 2 eines Patienten dargestellt; selbstverständlich und erfindungsgemäß sind aber auch andere Ausführungsformen, gerichtet auf die Behandlung anderer Knochen 2 von Mensch oder Tier, mit umfasst.

Das System 1 ist für ein an sich als erfindungsgemäß angesehenes Wirkungsprinzip zur Erreichung einer angestrebten Knochenmodifikation ausgelegt, bei dem ein flächig ausgedehntes Hub-/Druckelement 4 im oder am Knochen 2 positioniert und anschließend mit einer geeignet gewählten Kraft beaufschlagt wird. Im Falle eines angestrebten Knochenaufbaus wird das Hub-/Druckelement 4 dabei zwischen der Knochenhaut oder dem Periost 6 und dem eigentlichen Knochen 2 positioniert. Durch die auf das Hub-/Druckelement 4 ausgeübte äußere Kraft wird das Periost 6 in diesem Fall vom eigentlichen Knochen 2 abgehoben, so dass ein Freiraum zwischen Knochen 2 und Periost 6 entsteht. In diesen Freiraum können aus den Gefäßwänden des expandierten Gewebes Perzyten einwandern, die nachfolgend eine Knochenneubildung im geschaffenen Freiraum bewirken. Im anderen Fall, nämlich bei angestrebtem Knochenabbau, wird das Hub-/Druckelement 4 zwischen dem Periost 6 und dem umliegenden Bindegewebe positioniert und mit einer Kraft in Richtung zum Knochen 2 hin beaufschlagt, so dass Druck auf den Knochen 2 ausgeübt wird. Dieser bewirkt ein Zurückweichen des Knochens 2 und damit den angstrebten Knochenabbau.

Zur Übertragung oder Einleitung der vorgesehenen Kraft auf/in das Hub-/Drucke-lement 4 ist diesem ein Steuerelement 10 zugeordnet, das kraftseitig geeignet an das Hub-/Druckelement 4 angekoppelt oder ankoppelbar ist. Das Steuerelement 10 ist dabei, da es sich im Ausführungsbeispiel um die beabsichtigte Behandlung eines Kieferknochens 2 im Rahmen der Dentalchirurgie handelt, als Schiene ausgeführt, die geeignet auf das Zahnfleisch aufgelegt und endseitig an den Zähnen 12 des Patienten befestigt ist. Alternativ könnte das Steuerelement 10 auch außerhalb des Patienten positioniert und beispielsweise mittels eines Gurtsystems, einer geeigneten Gesichtsmaske oder einem beliebigen anderen Befestigungssystem am Kopf des Patienten befestigt sein.

Im Ausführungsbeispiel ist dabei eine magnetische Kraftübertragung zwischen Steuerelement 10 und Hub-/Druckelement 4 vorgesehen. Die das Steuerelement 10 bildende Schiene ist dabei geeignet ausgerüstet, um ein Magnetfeld im vorgesehenen Positionierungsbereich des Hub-/Druckelements 4 beim oder am Kieferknochen 2 zu erzeugen. Dazu kann das Steuerelement 10 eine Anzahl geeignet gewählter selektiv ansteuerbaren Elektromagnete umfassen; im Ausführungsbeispiel ist das Steuerelement 10 aber mit einer Anzahl von Permanentmagneten ausgerüstet. Bei einer Ausrüstung mit Elektromagneten kann durch elektrische Ansteuerung des Steuerrlements 10 das Magnetfeld im Bereich der Positionierung des Hub-/Druckelements 4 und damit die auf das Hub-/Druckelement 4 einwirkende und von diesem auf das Periost 6 übertragene Kraft auf besonders einfache Weise geeignet eingestellt und bedarfsweise nachgeführt, d. h. beispielsweise entsprechend einem festgestellten Behandlungserfolg neu eingestellt, werden.

Das System 1, umfassend das Hub-/Druckelement 4 und das zugeordnete Steuerelement 10, ist hinsichtlich der magnetischen Kopplung und Kraftübertragung geeignet für die angestrebte Schaffung des erwünschten Freiraums zwischen Periost 6 und Knochen 2 bzw. zwischen Periost 6 und Bindegewebe ausgelegt. Dazu sollte die vom Steuerelement 10 magnetisch auf das Hub-/Druckelement übertragene Kraft in etwa derart gewählt sein, dass pro Tag die sich ergebende Knochendistraktion etwa 0,5 mm beträgt. Im Ausführungsbeispiel ist das Hub-/Druckelement 4 auf Basis gekapselten oder mit Teflon ummantelten Esiens und somit ferromagnetisch ausgeführt. Die Magnetisierung oder Magnetisierbarkeit des Hub-/ Druckelements 4 ist dabei im Hinblick auf die angestrebte zu übertragende Kraft geeignet gewählt.

Zur Durchführung der vorgesehenen Behandlung wird das Hub-/Druckelement 4 vorübergehend zwischen Knochen 2 und Periost 6 bzw. zwischen Periost 6 und Bindegewebe implantiert. Hinsichtlich seiner Eigenschaften wie bespielsweise Flexibilität, Dicke, Durchlässigkeit oder dergleichen ist das Hub-/Druckelement 4 derart ausgestaltet, dass einerseits die gewünschte Platzierung besonders einfach möglich und andererseits durch eine adäquate Permeabilität die Reanastomosierung des Periosts 6 mit Gefäßen im Knochen 2 ermöglicht ist.

Insbesondere ist das Hub-/Druckelement 4 dabei mit besonders geringer Dicke, also so dünn wie möglich, ausgeführt. Damit ist sichergestellt, dass bei der Implantation nach Freilegung des Knochens 2 und Applikation des Hub-/Druckelements 4 das Periost 6 und das darüberliegende Weichgewebe spannungsfrei über dem Hub-/Druckelement 4 geschlossen werden kann. Dies ist für die Wundheilung wichtig. Andererseits ist die Dicke aber auch ausreichend groß gewählt, so eine ausreichende mechanische Stabilität zur Übertragung der vorgesehenen Kräfte gewährleistet ist.

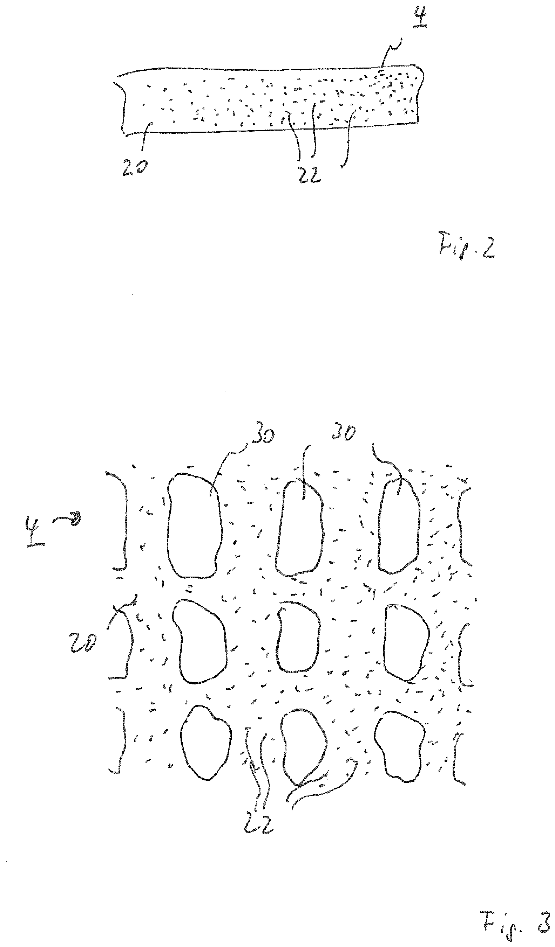

Um sich während der Behandlung, also beim Knochenaufbau von "klein nach groß" bzw. bei Knochenabbau umgekehrt, geeignet verändern und an die sich im Zuge der Behandlung ändernden Umgebungsbedingungen anpassen zu können, ist das Hub-/Druckelement 4 hinreichend flexibel, d. h. in der Form veränderbar, ausgeführt. Um dabei gleichermaßen zuverlässig eine flächig ausgedehnte Magnetisierbarkeit zu ermöglichen, ist das Hub-/Druckelement 4, wie dies der ausschnittsweise vergrößerten Darstellung im Querschnitt in FIG. 2 dargestellt ist, von einem Grundkörper 20 aus einem geeignet gewählten, insbesondere biokompatiblen und zudem auch ausreichend flexiblen Kunststoff gebildet. Der Grundkörper 20 bildet dabei eine Kunststoffmatrix, in die magnetische oder magnetisierbare Partikel 22 eingebettet sind. Durch diesen Aufbau sind die Partikel 22 im Sinne einer besonders guten Biokompatibiität oder Verträglichkeit in der Art einer gekapselten Ausführung vollständig in den Kunststoff-Grundköper 20 eingebettet. Zur Bereitstellung der gewünschten magnetischen Eigenschaften bestehen die Partikel 22 dabei aus Eisen.

Um während der Behandlung, also auch bei implantierem Hub-/Druckelement 4, einen Gefäßneuanschluß zwischen Gefäßen im Knochen 2 und im Periost 6 zu ermöglichen, ist das Hub-/Druckelement 4 perforiert und somit in der Fläche durchlässig ausgeführt. Dies ist besonders gut in der Draufsicht des Hub-/Druckelements 4 gemäß FIG. 3 erkennbar. Zur Bildung der Perforation ist der das Hub-/ Druckelement 4 bildende Grundkörper 20 flächig gesehen mit vergleichsweise groß gehaltenen Löchern 30 versehen, wobei der Grundkörper 20 zwischen diesen eine Art Gitter-Netz-Körper bildet. Alternativ könnten zur Bildung der gewünschten Perforation natürlich auch kleinere Löcher vorgesehen sein. Im Ausführungsbeispiel ist ein Verhältnis Perforation zu Fläche von etwa 50 % vorgesehen. Die durch die Größe der Löcher 30 definierte Porengröße der die Perforation bildenden Löcher 30 ist dabei zudem klein genug gewählt, um ein "Durchrutschen" des Periosts 6 zu vermeiden und eine gleichmäßige Elevation des gesamten Weichgewebes zu erreichen.

Durch die Formgebung des Grundkörpers 20 einerseits und den grundsätzlichen Aufbau als Matrixkörper mit eingebetteten magnetischen Partikeln 22 andererseits ergibt sich für das dargestellte Ausführungsbeispiel, dass das dargestellte Hub-/ Druckelement 4 eine über seine Fläche gesehen eine in der Art einer Gitterstruktur verteilte Magnetisierung bzw. Magnetisierbarkeit aufweist.

### Bezugszeichenliste

- 1: System
- 2: Knochen
- 4: Hub-/Druckelement
- 6: Periost
- 10: Steuerelement
- 12: Zahn
- 20: Grundkörper
- 22: Partikel
- 30: Loch

## Patentansprüche

1. System (1) zur Modifikation eines menschlichen oder tierischen Knochens (2), mit einem zur Einbringung zwischen die Knochenhaut (6) des zu behandelnden Knochens (2) und den darunterliegenden Knochen (2) oder das darüberliegende Bindegewebe vorgesehenen flächig ausgedehnten Hub-/Druckelement (4), das von einer Kunststoffmatrix gebildet ist, in die magnetische oder magnetisierbare Partikel (22) eingebettet sind, und mit einem diesem zugeordneten Steuerelement (10), wobei das Steuerelement (10) zur Ausübung einer magnetischen Druck- oder Zugkraft auf das Hub-/Druckelement (4) ausgelegt ist, die derart gewählt ist, dass die sich unter Berücksichtigung der Magnetisierung des Hub-/Druckelements (4) pro Tag ergebende Knochendistraktion etwa 0,5 mm beträgt.

2. System (1) nach Anspruch 1, dessen Hub-/Druckelement (4) permanent- oder ferromagnetisch ist.

3. System (1) nach Anspruch 1 oder 2, dessen Hub-/Druckelement (4) eine über seine Fläche gesehen im Wesentlichen konstante Magnetisierung bzw. Magnetisierbarkeit aufweist.

4. System (1) nach Anspruch 1 oder 2, dessen Hub-/Druckelement (4) eine über seine Fläche gesehen eine in der Art einer Gitterstruktur verteilte Magnetisierung bzw. Magnetisierbarkeit aufweist.

## Claims

1. System (1) for the modification of a human or animal bone (2) with a two-dimensionally extending lifting/pressing element (4) which is provided for introduction between the periosteum (6) of the bone (2) to be treated and the underlying bone (2) or the connective tissue above it, and is made of a plastic matrix in which magnetic or magnetisable particles (22) are embedded, and with a control element (10) assigned thereto, wherein the control element (10) is designed to apply a magnetic pressure or tensile form on the lifting/pressing element (4) that is selected in such a way that taking into consideration the magnetisation of the lifting/pressing element (4), the resulting bone distraction is around 0.5 mm per day.

2. System (1) according to claim 1, the lifting/pressing element (4) of which is permanently or ferromagnetic.

3. System (1) according to claim 1 or 2, the lifting/pressing element (4) of which, seen over its surface area, exhibits essentially constant magnetisation or magnetisability.

4. System (1) according to claim 1 or 2, the lifting/pressing element (4) of which, seen over its surface area, exhibits magnetisation or magnetisability distributed in the form of a grid structure.

## Revendications

1. Système (1) de modification d'un os humain ou animal (2), comportant un élément de levage/compression (4) s'étendant en surface et prévu pour être intégré entre le périoste (6) de l'os à traiter (2) et l'os sous-jacent (2) ou le tissu conjonctif situé au-dessus et qui est composé d'une matrice de matière plastique dans laquelle des particules magnétiques ou magnétisables (22) sont encastrées, et un élément de commande qui lui est associé(10), l'élément de commande (10) étant conçu pour exercer une force de compression ou de traction magnétique sur l'élément de levage/compression (4) qui est choisi de manière à ce que la distraction osseuse, en tenant compte de la magnétisation de l'élément de levage/compression (4), soit d'environ 0,5 mm par jour.

2. Système (1) selon la revendication 1, dont l'élément de levage/compression (4) est magnétique de manière permanente ou ferromagnétique.

3. Système (1) selon la revendication 1 ou 2, dont l'élément de levage/compression (4) présente une magnétisation ou une possibilité de magnétisation sensiblement constante, vu sur sa surface.

4. Système (1) selon la revendication 1 ou 2, dont l'élément de levage/compression (4) présente une magnétisation ou une possibilité de magnétisation répartie à la manière d'une structure tramée, vu sur sa surface.
